# EUROPÄISCHE PATENTSCHRIFT

(11) **EP 0 342 616 B1**
(45) Veröffentlichungstag und Bekanntmachung des Hinweises auf die Patenterteilung: **02.02.1994**
(21) Anmeldenummer: 89108794.2
(22) Anmeldetag: 16.05.1989
(51) Int. Cl.: C07C 255/27

(54) **Verfahren zur Herstellung von Aminocyanacetamid**
Process for the preparation of aminocyanacetamide
Procédé de préparation d'aminocyanacétamide

(30) Priorität: 17.05.1988 CH 1861/88
(43) Veröffentlichungstag der Anmeldung: 23.11.1989
(73) Patentinhaber: LONZA AG, CH-4002 Basel (CH)
(72) Erfinder: Mettler, Hans Peter, Dr., Brig-Glis Wallis (CH); Previdoli, Felix, Dr., Brig-Glis Wallis (CH)
(74) Vertreter: Weinhold, Peter, Dr.

(56) Entgegenhaltungen:
- EP-A- 0 298 261
- DE-A- 2 700 733

## Beschreibung

Die Erfindung betrifft ein neues, grosstechnisch einsetzbares Verfahren zur Herstellung von Aminocyanacetamid. Aminocyanacetamid ist ein interessantes Zwischenprodukt zur Herstellung von z.B. Imidazolen, Pyridazinen, Purinen oder Pteridinen.

Zu dessen Herstellung, bzw. zur Herstellung des Aminocyanessigesters, sind mehrere Verfahren bekannt.

Aus der DE-OS 27 00 733 geht ein Verfahren zur Herstellung von Aminocyanessigsäureethylester aus dem Hydroxyiminocyanessigsäureethylester durch Hydrierung mit Raney-Nickel hervor. Die danach erreichbare Ausbeute liegt bei 65%.
Neben der unbefriedigenden Ausbeute und der schlechten Qualität des isolierten Produktes ist von grossem Nachteil, dass der Katalysator nur mit relativ grossem Aufwand regenerierbar und rezirkulierbar ist. Für ein grosstechnisches Verfahren rechtfertigt sich ein solcher Aufwand nicht.

Von Logeman et al, Chem. and Ind. (1980), 541, ist bekannt, dass man den Hydroxyiminocyanessigsäureethylester mit Natriumdithionit und in Gegenwart von Ethanol als Lösungsmittel mit einer Ausbeute von 81% (Rohprodukt)in den Aminocyanessigsäureethylester umsetzen kann.
Nachteilig bei diesem Verfahren ist der entstehende Sulfatabfall sowie der Einsatz von feuergefährlichem Ether. Hinsichtlich der Entsorgungsproblematik von Salzabfällen wird, wo immer möglich, von solchen Verfahren Abstand genommen.

Schliesslich ist von Taylor et al, J.Am.Chem.Soc. 98, 2301 (1976) bekannt, Hydroxyiminocyanessigsäurebenzylester in Gegenwart von Aluminiumamalgan und Ether als Lösungsmittel und in einem zweiten Schritt mit Methansulfonsäure in einer Ausbeute von 53% in das Methansulfonat des Aminocyanessigsäurebenzylesters überzuführen.
Von der Entsorgungsproblematik des Quecksilberkatalysators her ist dieses Verfahren für eine technische Anwendung nicht mehr diskutabel.

Mit den gleichen Nachteilen behaftet ist das Verfahren von Smith et al, J.Am.Chem.Soc. 76, 6080 (1954). Darin gelangt man, ausgehend von Hydroxyiminocyanacetamid, durch Hydrierung mit Aluminiumamalgan direkt zu Aminocyanacetamid, mit einer Ausbeute von 59%.

Aufgabe der vorliegenden Erfindung ist es, ein Verfahren ohne die genannten Nachteile aufzuzeigen und mit dem man in der Lage ist, das Aminocyanacetamid, ausgehend von einem grosstechnisch verfügbaren Ausgangsprodukt, in technischem Massstab und auf möglichst umweltverträgliche Weise herzustellen.
Diese Aufgabe konnte gelöst werden mit einem Verfahren nach Patentanspruch 1.

Ausgangspunkt des Verfahrens sind die grosstechnisch verfügbaren Cyanessigsäureester. Geeignet für die erfindungsgemässe Umsetzung sind die C₁-C₄-alkylester, wie die Methyl-, Ethyl-, Propyl-, Butyl- oder tert.Butylester, oder die Arylalkylester, wie der Benzylester.

Die Cyanessigsäureester werden in einem ersten Schritt auf bekannte Weise, z.B. gemäss G. Duguay, J.Heterocycl.Chem. 17, 767 (1980) zum entsprechenden Hydroxyiminocyanessigsäureester nitrosiert. Als Nitrosierungsmittel werden üblicherweise Alkalinitrite, vor allem das Natriumnitrit, eingesetzt.
Die Reaktion erfolgt in sauren Medien.

Der resultierende Hydroxyiminocyanessigsäureester wird in der Regel dann in eine organischen Phase übergeführt, woraus er gegebenenfalls isoliert oder vorzugsweise direkt für die Folgestufe eingesetzt werden kann.

Die Folgestufe, die Hydrierung des Hydroxyiminocyanessigesters zum Aminocyanessigester, erfolgt mit Wasserstoff in Gegenwart eines Platinkatalysators. Geeignete Platinkatalysatoren sind Platin, das in Mengen von 1 bis 20 Gew.% auf Kohle, Aluminiumoxid, Siliciumdioxid, Bariumsulfat oder Calciumcarbonat als Trägermaterialien fein verteilt ist, oder Platinoxid. Bevorzugt wird Platin in einer Menge von 3 bis 10 Gew.% auf Kohle eingesetzt.
Zweckmässig wird der Platinkatalysator in Mengen von 3 bis 30 Gew.%, vorzugsweise 10 bis 15 Gew.%, bezogen auf den Hydroxyiminocyanessigester, verwendet.

Die Hydrierung erfolgt erfahrungsgemäss bei einem Druck von 1 bis 100 bar, besonders vorteilhaft bei 6 bis 10 bar, und bei Temperaturen von 0 bis 40°C, bevorzugt bei Raumtemperatur.

Es ist von Vorteil, in Gegenwart eines tiefsiedenden organischen Lösungsmittels, wie einem niederen aliphatischen Alkohol, z.B. Ethanol, wie einem niederen Carbonsäureester, z.B. Ethylacetat, oder einer niederen Carbonsäure, z.B. Essigsäure, zu arbeiten.

Jeweils abhängig von Druck, Temperatur und Katalysatormenge, kann die Hydrierung in einem Bereich von 0,5 bis 10 h variieren.

Ein weiterer Vorzug des erfindungsgemässen Verfahrens ist, dass der Platinhydrierkatalysator nach beendeter Reaktion rezirkuliert und für die nächste Hydrierung eingesetzt werden kann.

Der resultierende Aminocyanessigsäureester kann aus der Reaktionslösung isoliert werden, vorzugsweise wird aber direkt mit Ammoniak zum Endprodukt, zum Aminocyanacetamid, umgesetzt.

Die Umsetzung zum Aminocyanacetamid erfolgt in wässrigem Ammoniak, einer Konzentration von zweckmässig 10 bis 40 Gew.%, bei Temperaturen zwischen -20 bis 30°C, bevorzugt zwischen 0 bis 5°C.

Das Molverhältnis Ammoniak zu Aminocyanessigester wählt man zweckmässig zwischen 30:1 und 1:1, vorzugsweise zwischen 10:1 und 5:1.

Das Lösungsmittel entspricht weitgehend dem der Vorstufe. Es ist aber auch möglich, ohne zusätzliches Lösungsmittel, d.h. lediglich im wässrigen Ammoniak als Lösungsmittel, zu arbeiten.

Das resultierende Aminocyanacetamid kann nach üblicher Aufarbeitung in Ausbeuten von grösser als 70%, bezogen auf den Cyanessigsäureester, mit einem Gehalt von mehr als 98% erhalten werden.

Da bereits der Aminocyanessigsäureester ein beliebtes Zwischenprodukt für zahlreiche Synthesen ist, selber aber nicht sehr lagerstabil ist, besteht alternativ die Möglichkeit, nach Beendigung der Hydrierung der Reaktionslösung eine Säure zuzusetzen und den Aminocyanessigester in ein Salz überzuführen. Besonders geeignete Salze der Aminocyanessigsäureester sind die Tosylate, Oxalate oder Methansulfonate, besonders bevorzugt die Tosylate.
Diese Salze zeichnen sich aus durch ihre hohe Lagerstabilität, einfache Handhabung und die Möglichkeit der Herstellung in hoher Reinheit.

### Beispiel 1

### Herstellung von Hydroxyiminocyanessigsäureethylester aus Cyanessigsäureethylester

Zu 113,7 g (1,0 mol) Cyanessigsäureethylester und 83,2 g (1,2 mol) Natriumnitrit, gelöst in 900 ml Eiswasser, tropfte man bei 10°C während 30 min 83,0 g Eisessig. Diese Lösung wurde während 4 h bei 20°C gerührt, mit 100 ml konz. Salzsäure versetzt und mit 4 x 150 ml Ethylacetat extrahiert.
Die organische Phase wurde mit 4 x 50 ml Eiswasser neutralgewaschen, über Natriumsulfat getrocknet, auf 245 g eingeengt und 12 h bei 4°C stehengelassen.
Der ausgefallene Hydroxyiminocyanessigsäureethylester wurde abfiltriert, mit kaltem Ethylacetat gewaschen und getrocknet. Man erhielt 130,6 g Produkt in einer Ausbeute von 91,9%, bezogen auf Cyanessigsäureethylester mit einem Smp. von 129,5 bis 131°C.

| Elementaranalyse für C₅H₆N₂O₃: | | | |
|---|---|---|---|
| gef. | C = 41,75% | H = 4,25% | N = 19,11% |
| ber. | C = 42,26% | H = 4,26% | N = 19,71% |

### Beispiel 2

### Herstellung von Aminocyanessigsäuremethylester-tosylat aus Hydroxyiminocyanessigsäuremethylester

Eine Lösung von 18,0 g (0,14 mol) Hydroxyiminocyanessigsäuremethylester in 200 ml Ethanol wurde bei 10 bar Wasserstoffdruck und Raumtemperatur über 2,7 g Platin 5% auf Kohle hydriert. Das Reaktionsgemisch wurde filtriert, das Filtrat mit 26,9 g (0,14 mol) Toluolsulfonsäuremonohydrat versetzt, mit 750 ml Toluol vermischt, auf 340 g eingeengt und 12 h bei 4°C stehengelassen.
Das ausgefallene Aminocyanessigsäuremethylester-tosylat wurde abfiltriert, mit Toluol gewaschen und getrocknet.
Man erhielt 36,9 g Produkt in einer Ausbeute von 89,7%, bezogen auf Hydroxyiminocyanessigsäuremethylester, mit einem Smp. von 162,5 bis 164,5°C.

| Elementaranalyse für C₁₁H₁₄N₂O₅S: | | | |
|---|---|---|---|
| gef. | C = 45,80% | H = 4,92% | N = 9,65% |
| ber. | C = 46,18% | H = 4,93% | N = 9,79% |

### Beispiel 3

### Herstellung von Aminocyanessigsäureethylester-tosylat aus Hydroxyiminocyanessigsäureethylester

Eine Lösung von 20,0 g (0,14 mol) Hydroxyiminocyanessigsäureethylester in 200 ml Ethanol wurde bei 10 bar Wasserstoffdruck und Raumtemperatur über 2,6 g Platin 5% auf Kohle hydriert.
Das Reaktionsgemisch wurde filtriert, das Filtrat mit 26,9 g (0,14 mol) Toluolsulfonsäuremonohydrat versetzt, mit 1200 ml Toluol vermischt, auf 330 g eingeengt und 12 h bei 4°C stehengelassen.
Das ausgefallene Aminocyanessigsäureethylester-tosylat wurde abfiltriert, mit Toluol gewaschen und getrocknet.
Man erhielt 40,0 g Produkt in einer Ausbeute von 93,4%, bezogen auf Hydroxyiminocyanessigsäureethylester, Smp. 128 bis 130°C.
Durch Umkristallisation aus Ethylacetat isolierte man ein Produkt mit einem Smp. von 130 bis 131°C.

| Elementaranalyse für C₁₂H₁₆N₂O₅S: | | | |
|---|---|---|---|
| gef. | C = 47,44% | H = 5,42% | N = 9,21% |
| ber. | C = 48,00% | H = 5,37% | N = 9,33% |

### Beispiel 4

### Herstellung von Aminocyanessigsäurebenzylester-tosylat aus Hydroxyiminocyanessigsäurebenzylester

Eine Lösung von 20,4 g (0,1 mol) Hydroxyiminocyanessigsäurebenzylester in 200 ml Ethanol wurde bei 10 bar Wasserstoffdruck und Raumtemperatur über 3,1 g Platin 5% auf Kohle hydriert. Das Reaktionsgemisch wurde filtriert, das Filtrat mit 19,2 g (0,1 mol) Toluolsulfonsäuremonohydrat versetzt, auf 150 g eingeengt, mit 400 ml Toluol vermischt, auf 230 g eingeengt und 12 h bei 4°C stehengelassen.
Das ausgefallene Aminocyanessigsäurebenzylester-tosylat wurde abfiltriert, mit Toluol gewaschen und getrocknet.
Man erhielt 31,2 g Produkt in einer Ausbeute von 86,3%, bezogen auf Hydroxyiminocyanessigsäurebenzylester, Smp. 171 bis 172°C.
Durch Umkristallisation aus Isopropanol isolierte man ein Produkt mit einem Smp. von 173 bis 174°C.

| Elementaranalyse für C₁₇H₁₈N₂O₅S: | | | |
|---|---|---|---|
| gef. | C = 55,60% | H = 4,95% | N = 7,82% |
| ber. | C = 56,38% | H = 5,01% | N = 7,73% |

### Beispiel 5

### Herstellung von Aminocyanessigsäure-tert.-butylester-tosylat aus Hydroxyiminocyanessigsäure-tert.-butylester

Eine Lösung von 21,3 g (0,1 mol) Hydroxyiminocyanessigsäure-tert.-butylester in 200 ml Ethanol wurde bei 10 bar Wasserstoffdruck und Raumtemperatur über 3,2 g Platin 5% auf Kohle hydriert. Das Reaktionsgemisch wurde filtriert, das Filtrat mit 19,0 g (0,1 mol) Toluolsulfonsäuremonohydrat versetzt, mit 500 ml Toluol vermischt, auf 204 g eingeengt und 12 h bei 4°C stehengelassen.
Das ausgefallene Aminocyanessigsäure-tert.-butylester-tosylat wurde abfiltriert, mit Toluol gewaschen und getrocknet.
Man erhielt 16,2 g Produkt in einer Ausbeute von 51,1%, bezogen auf Hydroxyiminocyanessigsäure-tert.-butylester, mit einem Smp. von 124 bis 126°C.
Durch Umkristallisation aus Ethylacetat/Ethanol 3:1 isolierte man ein Produkt mit einem Smp. von 128 bis 129,5°C.

| Elementaranalyse für C₁₄H₂₀N₂O₅S: | | | |
|---|---|---|---|
| gef. | C = 51,15% | H = 6,21% | N = 8,65% |
| ber. | C = 51,24% | H = 6,14% | N = 8,54% |

### Beispiel 6

### Herstellung von Aminocyanessigsäureethylester-tosylat aus Cyanessigsäureethylester

Zu 17,1 g (0,15 mol) Cyanessigsäureethylester und 12,4 g (0,18 mol) Natriumnitrit, gelöst in 135 ml Eiswasser, tropfte man bei 10°C während 10 min 12,5 g (0,21 mol) Eisessig. Die Lösung wurde während 1 h bei 20°C gerührt, mit 15 ml konz. Salzsäure versetzt und mit 4 x 50 ml Ethylacetat extrahiert. Die organische Phase wurde mit 3 x 15 ml Eiswasser neutralgewaschen, über Natriumsulfat getrocknet und auf 200 g eingeengt. Diese Lösung hydrierte man bei 10 bar Wasserstoffdruck und Raumtemperatur über 3,2 g Pt/C (5%).
Das Reaktionsgemisch wurde filtriert, das Filtrat mit einer Lösung von 28,8 g (0,15 mol) Toluolsulfonsäuremonohydrat in 25 g Methanol versetzt, auf 120 g eingeengt, mit 200 ml Toluol vermischt, wieder auf 155 g eingeengt und 12 h bei 4°C stehengelassen. Das ausgefallene Aminocyanessigsäureethylester-tosylat wurde abfiltriert, mit Toluol gewaschen und getrocknet. Nach Umkristallisation aus Ethylacetat isolierte man 33,7 g Produkt, in einer Ausbeute von 74,9%, bezogen auf Cyanessigsäureethylester.

### Beispiel 7

### Herstellung von Aminocyanacetamid aus Hydroxyiminocyanessigsäuremethylester

Eine Lösung von 38,6 g (0,3 mol) Hydroxyiminocyanessigsäuremethylester in 200 ml Methanol wurde bei 10 bar Wasserstoffdruck und Raumtemperatur über 3,1 g Platin 5% auf Kohle hydriert. Das Reaktionsgemisch wurde filtriert, das Filtrat mit 15 ml Eiswasser versetzt und bei 0°C zu einer Lösung von 9,0 g Ammoniak in 25 g Methanol gegeben. Das Reaktionsgemisch wurde während 0,5 h bei 0°C gerührt.
Das ausgefallene Aminocyanacetamid wurde abfiltriert, mit Toluol gewaschen und getrocknet.
Man erhielt 18,4 g Produkt mit einem Smp. von 119,5 bis 120,5°C. Das Filtrat wurde auf 150 ml eingeengt, mit 450 ml Toluol vermischt, auf 300 ml eingeengt und 12 h bei 4°C stehengelassen.
Das ausgefallene Aminocyanacetamid wurde abfiltriert, mit Toluol gewaschen und getrocknet.
Man erhielt weitere 6,0 g Produkt mit einem Smp. von 112 bis 115°C.
Gesamtausbeute: 82,0%, bezogen auf Hydroxyiminocyanessigsäuremethylester.

| Elementaranalyse für C₃H₅N₃O: | | | |
|---|---|---|---|
| gef. | C = 36,2% | H = 5,1% | N = 40,5% |
| ber. | C = 36,4% | H = 5,1% | N = 42,4% |

### Beispiel 8

### Herstellung von Aminocyanacetamid aus Cyanessigsäuremethylester (Direktsynthese)

Zu 40,0 g (0,40 mol) Cyanessigsäuremethylester und 33,5 g (0,48 mol) Natriumnitrit, gelöst in 300 ml Wasser, tropfte man bei 10°C während 20 min 31,8 g (0,53 mol) Eisessig. Die Lösung wurde während 1 h bei 20°C gerührt, mit 50,6 g (0,5 mol) konz. Salzsäure versetzt und mit 6 x 50 ml Ethylacetat extrahiert. Die organische Phase wurde mit 2 x 50 ml Wasser neutralgewaschen, über Natriumsulfat getrocknet, auf 150 ml eingeengt und mit 100 ml Methanol vermischt. Diese Lösung hydrierte man bei 6 bis 10 bar Wasserstoffdruck und Raumtemperatur über 5,1 g Pt/C (5%). Das Reaktionsgemisch wurde anschliessend filtriert, mit 15 ml Wasser versetzt und bei 0 bis 5°C zu einer Lösung von 36,7 g (2,2 mol) Ammoniak in 150 ml Methanol gegeben. Nach 1 h wurde das ausgefallene Produkt abfiltriert, mit Methanol gewaschen und getrocknet.
Man erhielt 20,4 g Aminocyanacetamid mit einem Smp. von 121 bis 122°C. Das Filtrat wurde auf 50 g eingeengt, kühlgestellt und nochmals filtriert, wodurch weitere 8,7 g Produkt mit einem Smp. von 120 bis 121°C isoliert werden konnten.
Gesamtausbeute: 73,2%, bezogen auf eingesetzten Cyanessigsäuremethylester.

## Patentansprüche

1. Verfahren zur Herstellung von Aminocyanacetamid, dadurch gekennzeichnet, dass man einen Cyanessigsäure-(C₁-C₄)-alkylester oder einen -arylalkylester in Gegenwart eines Alkalinitrits zum entsprechenden Hydroxyiminocyanessigsäureester nitrosiert, diesen gegebenenfalls isoliert oder ohne zu isolieren direkt, in Gegenwart eines Platinhydrierkatalysators, mit Wasserstoff zum Aminocyanessigsäureester hydriert, diesen gegebenenfalls isoliert oder ohne zu isolieren schliesslich mit wässrigem Ammoniak zum Endprodukt umsetzt.

2. Verfahren nach Patentanspruch 1, dadurch gekennzeichnet, dass als Platinkatalysator Platinoxid oder Platin, aufgebracht in Mengen von 1 bis 20 Gew.% auf ein Trägermaterial wie Aluminiumoxid, Siliciumdioxid, Bariumsulfat, Calciumcarbonat oder Kohle, eingesetzt wird.

3. Verfahren nach einem oder beiden der Patentansprüche 1 und 2, dadurch gekennzeichnet, dass die Hydrierung bei einem Druck von 1 bis 100 bar und Temperaturen zwischen 0 und 40°C durchgeführt wird.

4. Verfahren nach einem oder mehreren der Patentansprüche 1-3, dadurch gekennzeichnet, dass die Hydrierung in Gegenwart von niederen aliphatischen Alkoholen, niederen aliphatischen Carbonsäureestern oder niederen aliphatischen Carbonsäuren als Lösungsmittel durchgeführt wird.

5. Verfahren nach einem oder mehreren der Patentansprüche 1-4, dadurch gekennzeichnet, dass die Umsetzung mit wässrigem Ammoniak bei Temperaturen zwischen -20 bis 30°C durchgeführt wird.

6. Verfahren nach einem oder mehreren der Patentansprüche 1-5, dadurch gekennzeichnet, dass für die Umsetzung mit wässrigem Ammoniak auf 1 Moläquivalent Aminocyanessigsäureester 1 bis 30 Moläquivalente Ammoniak eingesetzt werden.

## Claims

1. A process for the preparation of aminocyanacetamide, characterized in that a cyanoacetic acid-(C₁-C₄)-alkyl ester or a cyanoacetic acid-(C₁-C₄)-aryl alkyl ester is nitrated in the presence of an alkali nitrite to the corresponding hydroxyiminocyanoacetic ester which is - optionally isolated or directly without isolation - hydrated with hydrogen in the presence of a platinum hydrogenation catalyst to the aminocyanoacetic ester which is then - optionally isolated or without isolation - reacted with aqueous ammonia to the final product.

2. A process according to claim 1, characterized in that as platinum catalyst are employed platinum oxide or platinum, applied in quantities of 1 to 20% by weight, to a support such as aluminum oxide, silicon dioxide, barium sulfate, calcium carbonate or carbon.

3. A process according to one or both of claims 1 and 2, characterized in that the hydrogenation is carried out under a pressure of 1 to 100 bar and at temperatures of between 0 and 40°C.

4. A process according to one or more of claims 1 to 3, characterized in that the hydrogenation is carried out in the presence of lower aliphatic alcohols, lower aliphatic carboxylic acid esters or lower aliphatic carboxylic acids as solvents.

5. A process according to one or more of claims 1 to 4, characterized in that the reaction is carried out with aqueous ammonia at temperatures of between -20 and 30 °C.

6. A process according to one or more of claims 1 to 5, characterized in that for the reaction with aqueous ammonia are employed 1 to 30 mol equivalents of ammonia per mol equivalent of aminocyanoacetic ester.

## Revendications

1. Procédé pour la préparation d'aminocyanacétamide, caractérisé en ce que l'on nitrose un alkylester de l'acide cyanacétique (C₁-C₄) ou un arylalkylester de l'acide cyanacétique en présence d'un nitrite alcalin en un ester hydroxyiminocyanacétique correspondant, en ce que l'on isole éventuellement celui-ci ou sans l'isoler en ce que l'on procède à son hydrogénation directement en présence d'un catalyseur d'hydrogénation au platine, avec de l'hydrogène pour donner un ester aminocyanacétique et en ce que l'on transforme celui-ci éventuellement isolé ou sans l'isoler pour finir avec de l'ammoniaque aqueux pour donner le produit final.

2. Procédé selon la revendication 1, caractérisé en ce qu'en tant que catalyseur au platine, on met en oeuvre un oxyde de platine ou du platine dans les quantités de 1 à 20 % en poids sur un matériau support tel que de l'oxyde d'aluminium, du dioxyde de silicium, du sulfate de baryum, du carbonate de calcium ou du carbone.

3. Procédé selon l'une ou les deux des revendications 1 et 2, caractérisé en ce que l'hydrogénation s'effectue une pression de 1 à 100 bars et des températures entre 0 et 40°C.

4. Procédé selon l'une ou plusieurs des revendications 1 à 3, caractérisé en ce que l'hydrogénation s'effectue en présence d'alcools aliphatiques inférieurs, d'esters carboxiliques aliphatiques inférieurs ou d'acides carboxiliques inférieurs en tant que solvant.

5. Procédé selon l'une ou plusieurs des revendications 1 à 4, caractérisé en ce que l'on effectue la réaction avec de l'ammoniaque aqueuse à des températures entre -20 jusqu'à 30°C.

6. Procédé selon l'une ou plusieurs des revendications 1 à 5, caractérisé en ce que pour la réaction avec l'ammoniaque aqueuse on met en oeuvre sur un mole équivalent d'ester aminocyanacétique 1 jusqu'à 30 moles équivalents d'ammoniaque.
